# EUROPÄISCHE PATENTSCHRIFT

(11) **EP 1 716 102 B1**
(45) Veröffentlichungstag und Bekanntmachung des Hinweises auf die Patenterteilung: **23.07.2014**
(21) Anmeldenummer: 05701202.3
(22) Anmeldetag: 27.01.2005
(51) Int. Cl.: C07C 253/10

(54) **VERFAHREN ZUR HERSTELLUNG VON PENTENNITRILEN AUS N-BUTAN**
METHOD FOR THE PRODUCTION OF PENTENE NITRILES FROM N-BUTANE
PROCEDE POUR PRODUIRE DES PENTENES NITRILES A BASE DE N-BUTANE

(30) Priorität: 29.01.2004 DE 102004004697
(43) Veröffentlichungstag der Anmeldung: 02.11.2006
(73) Patentinhaber: BASF SE, 67056 Ludwigshafen (DE)
(72) Erfinder: BARTSCH, Michael, 67433 Neustadt (DE); BAUMANN, Robert, 68159 Mannheim (DE); HADERLEIN, Gerd, 67269 Grünstadt (DE); SCHINDLER, Götz-Peter, 68219 Mannheim (DE); JUNGKAMP, Tim, B-2950 Kapellen (BE); LUYKEN, Hermann, 67069 Ludwigshafen (DE); SCHEIDEL, Jens, 69493 Hirschberg (DE); BRODHAGEN, Andreas, B-3060 Bierbeeg (BE)
(86) Internationale Anmeldenummer: PCT/EP2005/000773
(87) Internationale Veröffentlichungsnummer: WO 2005/073166

(56) Entgegenhaltungen:
- WO-A-99/07671
- GB-A- 628 686

## Beschreibung

Die vorliegende Erfindung betrifft ein Verfahren zur Hydrocyanierung von 1,3-Butadien an mindestens einem Nickel(0)-Komplex mit phosphorhaltigen Liganden als Katalysator.

Adipodinitril ist ein wichtiges Ausgangsprodukt in der Nylonherstellung, das durch zweifache Hydrocyanierung von 1,3-Butadien erhalten wird. In einer ersten Hydrocyanierung wird 1,3-Butadien zu 3-Pentennitril hydrocyaniert. In einer zweiten, sich anschließenden Hydrocyanierung wird 3-Pentennitril mit Cyanwasserstoff zu Adipodinitril umgesetzt. Beide Hydrocyanierungen werden durch Nickel(0)-Komplexe mit phosphorhaltigen Liganden katalysiert.

Für die Ausübung dieses Verfahrens zur Herstellung von Adipodinitril sind die Kosten und die Herkunft der genutzten Einsatzstoffe von entscheidender Bedeutung, da sie im Allgemeinen 70 % der Herstellungskosten ausmachen.

In der Adipodinitrilsynthese aus 1,3-Butadien und Cyanwaserstoff ist der Einsatz von reinem 1,3-Butadien in der Hydrocyanierungsreaktion unvorteilhaft, da 1,3-Butadien für gewöhnlich durch eine aufwendige Extraktion aus dem C₄-Schnitt von Steamcrackern abgetrennt wird, siehe beispielsweise Weissermehl, Arpe; Industrielle Organische Chemie; Seite 119 ff.; Wiley-VCH 1998.

Zur Einsparung von Extraktionskosten beschreibt die DE 196 52 273, dass ein roher C₄-Crackerschnitt anstelle von reinem 1,3-Butadien in der Hydrocyanierung eingesetzt werden kann. Dieser C₄-Crackerschnitt enthält im Allgemeinen 40 % 1,3-Butadien, 5 % Alkine und Allene sowie 55 % Mono- und Polyolefine. Während sich die Olefine in der Hydrocyanierung weitgehend inert verhalten, müssen die Alkine und Allene, z. B. durch eine zusätzliche Partialhydrierung, vor der Hydrocyanierung aus dem Gemisch entfernt werden, da es ansonsten zu einer Bildung unerwünschter Nebenprodukte und zur Inhibierung des Katalysators kommt. Aufgrund des hohen Anteils von ungefähr 60 % an Inertkomponenten ist die Raum-Zeit-Ausbeute bei Einsatz dieser partialhydrierten C₄-Crackerschnitte gegenüber dem Einsatz von reinem 1,3-Butadien signifikant erniedrigt.

Aus WO-A-99/07671 ist ein Verfahren zur Hydrocyanierung eines C₄ -Schrittes enthaltend 40.50 Vol.-% 1,3-Butadien und 2.90 Vol.-% n-Butadien an einem Ni(0) enthaltenden Katalysator mit einem 1,1'-Bis(diphenyl-phosphino) ferrocen Liganden bekannt.

Die Aufgabe der vorliegenden Erfindung ist es somit, ein Verfahren zur Hydrocyanierung von 1,3-Butadien an mindestens einem Katalysator bereit zu stellen, bei dem ein unaufwendig und kostengünstig zu erhaltener 1,3-Butadien-haltiger Eduktstrom verwendet werden kann.

Die Lösung dieser Aufgabe geht aus von einem Verfahren gemäß Anspruch 1 zur Hydrocyanierung von 1,3-Butadien an mindestens einem Nickel(0)-Komplex mit phosphorhaltigen Liganden als Katalysator. Das erfindungsgemäße Verfahren ist dann dadurch gekennzeichnet, dass das 1,3-Butadien in einer Mischung mit n-Butan eingesetzt wird.

Erfindungsgemäß wurde gefunden, dass eine Mischung aus 1,3-Butadien und n-Butan in die Hydrocyanierung zu 3-Pentennitril eingesetzt werden kann. Diese Mischung kann gegebenenfalls zusätzlich 2-Buten enthalten.

Die als Edukt für das erfindungsgemäße Verfahren verwendete Mischung enthält vorzugsweise 60 bis 90 Vol.-%, besonders bevorzugt 65 bis 85 Vol.%, insbesondere 70 bis 80 Vol.%, 1,3-Butadien, jeweils bezogen auf 1,3-Butadien und n-Butan. Darüber hinaus enthält die in dem erfindungsgemäßen Verfahren verwendete Mischung vorzugsweise 40 bis 10 Vol.-%, besonders bevorzugt 35 bis 15 Vol.%, insbesondere 30 bis 20 Vol.%, n-Butan, jeweils bezogen auf 1,3-Butadien und n-Butan.

Im erfindungsgemäßen Verfahren wird die Mischung, die in dem erfindungsgemäßen Verfahren zur Hydrocyanierung eingesetzt wird, als Wertproduktstrom e gemäß der prioritätsälteren, nicht vorveröffentlichten deutschen Patentanmeldung DE 103 61 822.8 der BASF AG, die ein Verfahren zur Herstellung von 1,3-Butadien betrifft, nach den folgenden Verfahrensschritten erhalten:
A) Bereitstellung eines n-Butan enthaltenden Einsatzgasstroms a;
B) Einspeisung des n-Butan enthaltenden Einsatzgasstroms a in mindestens eine erste Dehydrierzone und nicht-oxidative katalytische Dehydrierung von n-Butan, wobei ein Produktgasstrom b enthaltend n-Butan, 1-Buten, 2-Buten, Butadien, Wasserstoff, leichtsiedende Nebenbestandteile und gegebenenfalls Wasserdampf erhalten wird;
C) Einspeisung des Produktgasstroms b der nicht-oxidativen katalytischen Dehydrierung und eines sauerstoffhaltigen Gases in mindestens eine zweite Dehydrierzone und oxidative Dehydrierung von 1-Buten und 2-Buten, wobei ein Produktgasstrom c enthaltend n-Butan, 2-Buten, Butadien, Wasserstoff, leichtsiedende Nebenbestandteile und Wasserdampf erhalten wird, welcher einen höheren Gehalt an Butadien als der Produktgasstrom b aufweist;
D) Abtrennung von Wasserstoff, leichtsiedenden Nebenbestandteile und von Wasserdampf, wobei ein C₄-Produktgasstrom d im Wesentlichen bestehend aus n-Butan, 2-Buten und Butadien erhalten wird;
E) Einspeisung des C₄-Produktgasstroms d in eine Destillationszone und Abtrennung eines Butadien/n-Butan-Gemischs als Wertproduktstrom e.

Dieses Verfahren zur Herstellung des Wertproduktstromes e zeichnet sich durch eine besonders effektive Ausnutzung der Rohstoffe aus. So werden Verluste des Rohstoffs n-Butan durch Rückführung von nicht umgesetztem n-Butan in die Dehydrierung minimiert. Durch die Kopplung von nicht-oxidativer katalytischer Dehydrierung und oxidativer Dehydrierung wird eine hohe Butadien-Ausbeute erzielt.

In einem ersten Verfahrensteil A wird ein n-Butan enthaltender Einsatzgasstrom a bereitgestellt. Üblicherweise wird dabei von an n-Butan reichen Gasgemischen wie liquefied petroleum gas (LPG) als Rohstoff ausgegangen. LPG enthält im Wesentlichen gesättigte C₂-C₅-Kohlenwasserstoffe. Daneben enthält es auch Methan und Spuren von C₆⁺-Kohlenwasserstoffen. Die Zusammensetzung von LPG kann stark schwanken. Vorteilhafter Weise enthält das eingesetzte LPG mindestens 10 Gew.% Butane.

Alternativ kann ein veredelter C₄-Strom aus Crackern oder Raffinerien eingesetzt werden.

In einer Variante umfasst die Bereitstellung des n-Butan enthaltenden Dehydrier-Einsatzgasstromes die Schritte
(A1) Bereitstellung eines liquefied petroleum gas (LPG)-Stroms,
(A2) Abtrennung von Propan und gegebenenfalls Methan, Ethan und C₅⁺-Kohlenwasserstoffen (hauptsächlich Pentane, daneben Hexane, Heptane, Benzol, Toluol) aus dem LPG-Strom, wobei ein Butane (n-Butan und Isobutan) enthaltender Strom erhalten wird,
(A3) Abtrennung von Isobutan aus dem Butane enthaltenden Strom, wobei der n-Butan enthaltende Einsatzgasstrom erhalten wird, und gegebenenfalls Isomerisierung des abgetrennten Isobutans zu einem n-Butan/Isobutan-Gemisch und Rückführung des n-Butan/Isobutan-Gemischs in die Isobutan-Abtrennung.

Die Abtrennung von Propan und gegebenenfalls Methan, Ethan und C₅⁺-Kohlenwasserstoffen erfolgt beispielsweise in einer oder mehreren üblichen Rektifizierkolonnen. Beispielsweise können in einer ersten Kolonne Leichtsieder (Methan, Ethan, Propan) über Kopf und in einer zweiten Kolonne Schwersieder (C₅⁺-Kohlenwasserstoffe) am Kolonnensumpf abgetrennt werden. Es wird ein Butane (n-Butan und Isobutan) enthaltender Strom erhalten, aus dem Isobutan beispielsweise in einer üblichen Rektifizierkolonne abgetrennt wird. Der verbleibende, n-Butan enthaltende Strom wird als Einsatzgasstrom für die nachfolgende Butan-Dehydrierung eingesetzt.

Der abgetrennte Isobutan-Strom wird vorzugsweise einer Isomerisierung unterworfen. Dazu wird der Isobutan enthaltende Strom in einen Isomerisierungsreaktor eingespeist. Die Isomerisierung von Isobutan zu n-Butan kann wie in GB-A 2 018 815 beschrieben durchgeführt werden. Es wird ein n-Butan/lsobutan-Gemisch erhalten, das in die n-Butan/Isobutan-Trennkotonne eingespeist wird.

Der abgetrennte Isobutan-Strom kann auch einer weiteren Verwendung zugeführt werden, beispielsweise zur Herstellung von Methacrylsäure, Polyisobuten oder Methyl-tert.-butylether eingesetzt werden.

In einem Verfahrensteil B wird der n-Butan enthaltende Einsatzgasstrom in eine Dehydrierzone eingespeist und einer nicht-oxidativen katalytischen Dehydrierung unter-worfen. Dabei wird n-Butan in einem Dehydrierreaktor an einem dehydrieraktiven Katalysator teilweise zu 1-Buten und 2-Buten dehydriert, wobei auch Butadien gebildet wird. Daneben fallen Wasserstoff und in geringen Mengen Methan, Ethan, Ethen, Propan und Propen an. Je nach Fahrweise der Dehydrierung können außerdem Kohlenstoffoxide (CO, CO₂), Wasser und Stickstoff im Produktgasgemisch der nicht-oxidativen katalytischen n-Butan-Dehydrierung enthalten sein. Daneben liegt im Produktgasgemisch nicht umgesetztes n-Butan vor.

Die nicht-oxidative katalytische n-Butan-Dehydrierung kann mit oder ohne sauerstoffhaltigem Gas als Co-Feed durchgeführt werden.

Ein Merkmal der nicht-oxidativen Fahrweise (Dehydrierung unter Bildung von freiem Wasserstoff) gegenüber einer oxidativen Fahrweise ist das Vorhandensein von Wasserstoff im Austragsgas. Bei der oxidativen Dehydrierung wird kein freier Wasserstoff in wesentlichen Mengen gebildet.

Die nicht-oxidative katalytische n-Butan-Dehydrierung kann grundsätzlich in allen aus dem Stand der Technik bekannten Reaktortypen und Fahrweisen durchgeführt werden. Eine vergleichsweise ausführliche Beschreibung von erfindungsgemäß geeigneten Dehydrierverfahren enthält auch "Catatytica® Studies Division, Oxidative Dehydrogenation and Alternative Dehydrogenation Processes" (Study Number 4192 OD, 1993, 430 Ferguson Drive, Mountain View, California, 94043-5272, USA).

Eine geeignete Reaktorform ist der Festbettrohr- oder Rohrbündelreaktor. Bei diesen befindet sich der Katalysator (Dehydrierungskatalysator und, bei Arbeiten mit Sauerstoff als Co-Feed, gegebenenfalls spezieller Oxidationskatalysator) als Festbett in einem Reaktionsrohr oder in einem Bündel von Reaktionsrohren. Die Reaktionsrohre werden üblicherweise dadurch indirekt beheizt, dass in dem die Reaktionsrohre umgebenden Raum ein Gas, z.B. ein Kohlenwasserstoff wie Methan, verbrannt wird. Günstig ist es dabei, diese indirekte Form der Aufheizung lediglich auf den ersten ca. 20 bis 30% der Länge der Festbettschüttung anzuwenden und die verbleibende Schüttungslänge durch die im Rahmen der indirekten Aufheizung freigesetzte Strahlungswärme auf die erforderliche Reaktionstemperatur aufzuheizen. Übliche Reaktionsrohr-Innendurchmesser betragen etwa 10 bis 15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 300 bis 1200°C, vorzugsweise im Bereich von 500 bis 1000°C. Der Arbeitsdruck liegt üblicherweise zwischen 0,5 und 8x 10⁵ Pa, häufig zwischen 1 und 2 x 10⁵ Pa bei Verwendung einer geringen Wasserdampfverdünnung (analog dem Linde-Verfahren zur Propan-Dehydrierung), aber auch zwischen 3 und 8 bar bei Verwendung einer hohen Wasserdampfverdünnung (analog dem so genannten "steam active reforming process" (STAR-Prozess) zur Dehydrierung von Propan oder Butan von Phillips Petroleum Co., siehe US 4,902,849, US 4,996,387 und US 5,389,342). Typische Katalysatorbelastungen (GHSV) liegen bei 500 bis 2000 h⁻¹, bezogen auf eingesetzten Kohlenwasserstoff. Die Katalysatorgeometrie kann beispielsweise kugelförmig oder zylindrisch (hohl oder voll) sein.

Die nicht-oxidative katalytische n-Butan-Dehydrierung kann auch, wie in Chem. Eng. Sci. 1992 b, 47 (9-11) 2313 beschrieben, heterogen katalysiert im Wirbelbett durchgeführt werden. Zweckmäßigerweise werden dabei zwei Wirbelbetten nebeneinander betrieben, von denen sich eines in der Regel im Zustand der Regenerierung befindet. Der Arbeitsdruck beträgt typischerweise 1 bis 2x 10⁵ Pa, die Dehydriertemperatur in der Regel 550 bis 600°C. Die für die Dehydrierung erforderliche Wärme wird dabei in das Reaktionssystem eingebracht, indem der Dehydrierkatalysator auf die Reaktionstemperatur vorerhitzt wird. Durch die Zumischung eines Sauerstoff enthaltenden Co-Feeds kann auf die Vorerhitzer verzichtet werden, und die benötigte Wärme direkt im Reaktorsystem durch Verbrennung von Wasserstoff und/oder Kohlenwasserstoffen in Gegenwart von Sauerstoff erzeugt werden. Gegebenenfalls kann zusätzlich ein Wasserstoff enthaltender Co-Feed zugemischt werden.

Die nicht-oxidative katalytische n-Butan-Dehydrierung kann mit oder ohne Sauerstoffhaltigem Gas als Co-Feed in einem Hordenreaktor durchgeführt werden. Dieser enthält ein oder mehrere aufeinander folgende Katalysatorbetten. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im Allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von konzentrisch angeordneten zylindrischen Gitterrosten angeordnet. Ein Schachtofenreaktor entspricht einer Horde. Die Durchführung der Dehydrierung in einem einzelnen Schachtofenreaktor entspricht einer bevorzugten Ausführungsform, wobei mit sauerstoffhaltigem Co-Feed gearbeitet werden kann. In einer weiteren bevorzugten Ausführungsform wird die Dehydrierung in einem Hordenreaktor mit 3 Katalysatorbetten durchgeführt. Bei einer Fahrweise ohne sauerstoffhaltigem Gas als Co-Feed wird das Reaktionsgasgemisch im Hordenreaktor auf seinem Weg von einem Katalysatorbett zum nächsten Katalysatorbett einer Zwischenerhitzung unterworfen, z.B. durch Überleiten über mit heißen Gasen erhitzte Wärmeaustauscherflächen oder durch Durchleiten durch mit heißen Brenngasen beheizte Rohre.

In einer bevorzugten Ausführungsform des erfindungsgemäßen Verfahrens wird die nicht-oxidative katalytische n-Butan-Dehydrierung autotherm durchgeführt. Dazu wird dem Reaktionsgasgemisch der n-Butan-Dehydrierung in mindestens einer Reaktionszone zusätzlich Sauerstoff zugemischt und der in dem Reaktionsgasgemisch enthaltene Wasserstoff und/oder Kohlenwasserstoff zumindest teilweise verbrannt, wodurch zumindest ein Teil der benötigten Dehydrierwärme in der mindestens einen Reaktionszone direkt in dem Reaktionsgasgemisch erzeugt wird.

Im allgemeinen wird die Menge des dem Reaktionsgasgemisch zugesetzten sauerstoffhaltigen Gases so gewählt, dass durch die Verbrennung von im Reaktionsgasgemisch vorhandenen Wasserstoff und gegebenenfalls von im Reaktionsgasgemisch vorliegenden Kohlenwasserstoffen und/oder von in Form von Koks vorliegendem Kohlenstoff die für die Dehydrierung des n-Butans benötigte Wärmemenge erzeugt wird. Im Allgemeinen beträgt die insgesamt zugeführte Sauerstoffmenge, bezogen auf die Gesamtmenge des Butans, 0,001 bis 0,5 mol/mol, bevorzugt 0,005 bis 0,2 mol/mol, besonders bevorzugt 0,05 bis 0,2 mol/mol. Sauerstoff kann entweder als reiner Sauerstoff oder als sauerstoffhaltiges Gas im Gemisch mit Inertgasen, beispielsweise in Form von Luft, eingesetzt werden. Die Inertgase und die resultierenden Verbrennungsgase wirken im Allgemeinen zusätzlich verdünnend und fördern damit die heterogen katalysierte Dehydrierung.

Der zur Wärmeerzeugung verbrannte Wasserstoff ist der bei der katalytischen n-Butan-Dehydrierung gebildete Wasserstoff sowie gegebenenfalls dem Reaktionsgasgemisch als wasserstoffhaltiges Gas zusätzlich zugesetzter Wasserstoff. Vorzugsweise sollte soviel Wasserstoff zugegen sein, dass das Molverhältnis H₂/O₂ im Reaktionsgasgemisch unmittelbar nach der Einspeisung von Sauerstoff 1 bis 10, bevorzugt 2 bis 5 mol/mol beträgt. Dies gilt bei mehrstufigen Reaktoren für jede Zwischeneinspeisung von sauerstoffhaltigem und gegebenenfalls wasserstoffhaltigem Gas.

Die Wasserstoffverbrennung erfolgt katalytisch. Der eingesetzte Dehydrierungskatalysator katalysiert im Allgemeinen auch die Verbrennung der Kohlenwasserstoffe und von Wasserstoff mit Sauerstoff, so dass grundsätzlich kein von diesem verschiedener spezieller Oxidationskatalysator erforderlich ist. In einer Ausführungsform wird in Gegenwart eines oder mehrerer Oxidationskatalysatoren gearbeitet, die selektiv die Verbrennung von Wasserstoff zu Sauerstoff in Gegenwart von Kohlenwasserstoffen katalysieren. Die Verbrennung dieser Kohlenwasserstoffe mit Sauerstoff zu CO, CO₂ und Wasser läuft dadurch nur in untergeordnetem Maße ab. Vorzugsweise liegen der Dehydrierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Katalysator, der selektiv die Oxidation von Wasserstoff katalysiert, an den Stellen angeordnet, an denen höhere Sauerstoffpartialdrucke herrschen als an anderen Stellen des Reaktors, insbesondere in der Nähe der Einspeisungsstelle für das sauerstoffhaltige Gas. Die Einspeisung von sauerstoffhaltigem Gas und/oder wasserstoffhaltigem Gas kann an einer oder mehreren Stelle des Reaktors erfolgen.

In einer Ausführungsform erfolgt eine Zwischeneinspeisung von sauerstoffhaltigem Gas und von wasserstoffhaltigem Gas vor jeder Horde eines Hordenreaktors. In einer weiteren Ausführungsform des erfindungsgemäßen Verfahrens erfolgt die Einspeisung von sauerstoffhaltigem Gas und von wasserstoffhaltigem Gas vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem Dehydrierungskatalysator. In einer weiteren Ausführungsform ist kein spezieller Oxidationskatalysator vorhanden. Die Dehydriertemperatur beträgt im allgemeinen 400 bis 1100 °C, der Druck im letzten Katalysatorbett des Hordenreaktors im Allgemeinen 0,2 bis 5x 10⁵ Pa, bevorzugt 1 bis 3x 10⁵ Pa. Die Belastung (GHSV) beträgt im allgemeinen 500 bis 2000 h⁻¹, bei Hochlastfahrweise auch bis zu 100 000 h⁻¹, bevorzugt 4000 bis 16 000 h⁻¹.

Ein bevorzugter Katalysator, der selektiv die Verbrennung von Wasserstoff katalysiert, enthält Oxide und/oder Phosphate, ausgewählt aus der Gruppe bestehend aus den Oxiden und/oder Phosphaten von Germanium, Zinn, Blei, Arsen, Antimon oder Bismut. Ein weiterer bevorzugter Katalysator, der die Verbrennung von Wasserstoff katalysiert, enthält ein Edelmetall der VIII. und/oder I. Nebengruppe.

Die eingesetzten Dehydrierungskatalysatoren weisen im Allgemeinen einen Träger und eine Aktivmasse auf. Der Träger besteht dabei in der Regel aus einem wärmebeständigen Oxid oder Mischoxid. Bevorzugt enthalten die Dehydrierungskatalysatoren ein Metalloxid, das ausgewählt ist aus der Gruppe bestehend aus Zirkondioxid, Zinkoxid, Aluminiumoxid, Siliciumdioxid, Titandioxid, Magnesiumoxid, Lanthanoxid, Ceroxid und deren Gemischen, als Träger. Bei den Gemischen kann es sich um physikalische Mischungen oder auch um chemische Mischphasen wie Magnesium- oder Zinkaluminiumoxid-Mischoxide handeln. Bevorzugte Träger sind Zirkondioxid und/oder Siliziumdioxid, besonders bevorzugt sind Gemische aus Zirkondioxid und Siliziumdioxid.

Die Aktivmasse der Dehydrierungskatalysatoren enthalten im allgemeinen ein oder mehrere Elemente der VIII. Nebengruppe, bevorzugt Platin und/oder Palladium, besonders bevorzugt Platin. Darüber hinaus können die Dehydrierungskatalysatoren ein oder mehrere Elemente der I. und/oder II. Hauptgruppe aufweisen, bevorzugt Kalium und/oder Cäsium. Weiterhin können die Dehydrierungskatalysatoren ein oder mehrere Elemente der III. Nebengruppe einschließlich der Lanthaniden und Actiniden enthalten, bevorzugt Lanthan und/oder Cer. Schließlich können die Dehydrierungskatalysatoren ein oder mehrere Elemente der III. und/oder IV. Hauptgruppe aufweisen, bevorzugt ein oder mehrere Elemente aus der Gruppe bestehend aus Bor, Gallium, Silizium, Germanium, Zinn und Blei, besonders bevorzugt Zinn.

In einer bevorzugten Ausführungsform enthält der Dehydrierungskatalysator mindestens ein Element der VIII. Nebengruppe, mindestens ein Element der I. und/oder II. Hauptgruppe, mindestens ein Element der III. und/oder IV. Hauptgruppe und mindestens ein Element der III. Nebengruppe einschließlich der Lanthaniden und Actiniden.

Beispielsweise können erfindungsgemäß alle Dehydrierkatalysatoren eingesetzt werden, die in den WO 99/46039, US 4,788,371, EP-A 705 136, WO 99/29420, US 5,220,091, US 5,430,220, US 5,877,369, EP 0 117 146, DE-A 199 37 106, DE-A 199 37 105 und DE-A 199 37 107 offenbart werden. Besonders bevorzugte Katalysatoren für die vorstehend beschriebenen Varianten der autothermen n-Butan-Dehydrierung sind die Katalysatoren gemäß den Beispiel 1, 2, 3 und 4 der DE-A 199 37 107.

Die n-Butan-Dehydrierung wird bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit der Katalysatoren erhöht wird. Dabei werden die organischen Ablagerungen in Kohlenmonoxid, Kohlendioxid und gegebenenfalls Wasser umgewandelt.

Der Dehydrierungskatalysator kann in an sich bekannter Weise regeneriert werden. So kann dem Reaktionsgasgemisch Wasserdampf zugesetzt werden oder von Zeit zu Zeit ein Sauerstoff enthaltendes Gas bei erhöhter Temperatur über die Katalysatorschüttung geleitet werden und der abgeschiedene Kohlenstoff abgebrannt werden. Durch die Verdünnung mit Wasserdampf wird das Gleichgewicht zu den Produkten der Dehydrierung verschoben. Gegebenenfalls wird der Katalysator nach der Regenerierung mit einem wasserstoffhaltigen Gas reduziert.

Bei der nicht-oxidativen katalytischen n-Butan-Dehydrierung wird ein Gasgemisch erhalten, das neben Butadien 1-Buten, 2-Buten und nicht umgesetztem n-Butan Nebenbestandteile enthält. Übliche Nebenbestandteile sind Wasserstoff, Wasserdampf, Stickstoff, CO und CO₂, Methan, Ethan, Ethen, Propan und Propen. Die Zusammensetzung des die erste Dehydrierzone verlassenden Gasgemischs kann abhängig von der Fahrweise der Dehydrierung stark variieren. So weist bei Durchführung der bevorzugten autothermen Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

Der Produktgasstrom der nicht-oxidativen autothermen n-Butan-Dehydrierung enthält typischerweise 0,1 bis 15 Vol.-% Butadien, 1 bis 15 Vol.% 1-Buten, 1 bis 25 Vol.% 2-Buten (cis/trans-2-Buten), 20 bis 70 Vol.-% n-Butan, 1 bis 70 Vol.-% Wasserdampf, 0 bis 10 Vol.-% leichtsiedende Kohlenwasserstoffe (Methan, Ethan, Ethen, Propan und Propen), 0,1 bis 40 Vol.% Wasserstoff, 0 bis 70 Vol.-% Stickstoff und 0 bis 5 Vol.-% Kohlenstoffoxide.

Der die erste Dehydrierzone verlassende Produktgasstrom b wird bevorzugt in zwei Teilströme aufgetrennt, wobei nur einer der beiden Teilströme den weiteren Verfahrensteilen C bis G unterworfen wird und der zweite Teilstrom in die erste Dehydrierzone zurückgeführt werden kann. Eine entsprechende Verfahrensweise ist in DE-A 102 11 275 beschrieben. Es kann jedoch auch der gesamte Produktgasstrom b der nicht-oxidativen katalytischen n-Butan-Dehydrierung den weiteren Verfahrensteilen C bis F unterworfen werden.

Der nicht-oxidativen katalytischen Dehydrierung wird erfindungsgemäß eine oxidative Dehydrierung (Oxidehydrierung) als Verfahrensteil C nachgeschaltet. Dabei werden im Wesentlichen 1-Buten und 2-Buten zu 1,3-Butadien dehydriert, wobei 1-Buten im Allgemeinen nahezu vollständig abreagiert.

Diese kann grundsätzlich in allen aus dem Stand der Technik bekannten Reaktortypen und Fahrweisen durchgeführt werden, wie beispielsweise im Wirbelbett, im Hordenofen, im Festbettrohr- oder -rohrbündelreaktor oder im Plattenwärmetauscherreaktor. Ein Plattenwärmetauscherreaktor ist beispielsweise in DE-A 199 52 964 beschrieben. Zur Durchführung der oxidativen Dehydrierung wird ein Gasgemisch benötigt, welches ein molares Sauerstoff : n-Butene-Verhältnis von mindestens 0,5 aufweist. Bevorzugt wird bei einem Sauerstoff : n-Butene-Verhältnis von 0,55 bis 50 gearbeitet. Zur Einstellung dieses Wertes wird in der Regel das aus der nicht-oxidativen katalytischen Dehydrierung stammende Produktgasgemisch mit Sauerstoff oder einem sauerstoffhaltigem Gas, beispielsweise Luft, vermischt. Das erhaltene sauerstoffhaltige Gasgemisch wird dann der Oxidehydrierung zugeführt.

Die für die Oxidehydrierung besonders geeigneten Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten aus der 1. bis 15. Gruppe des Periodensystems, wie beispielsweise Kalium, Magnesium, Zirkon, Chrom, Nickel, Cobalt, Cadmium, Zinn, Blei, Germanium, Lanthan; Mangan, Wolfram, Phosphor, Cer, Aluminium oder Silizium.

Geeignete Katalysatoren und deren Herstellung sind beispielsweise beschrieben in US 4,423,281 (Mo₁₂BiNi₈Pb_{0,5}Cr₃K₀,₂Ox und Mo₁₂Bi_{b}Ni₇Al₃Cr₀,₅K_{0,5}Oₓ), US 4,336,409 (Mo₁₂BiNi₆Cd₂Cr₃P_{0,5}Oₓ), DE-A 26 00 128 (Mo₁₂BiNi_{0,5}Cr₃P_{0,5}Mg_{7,5}K₀,₁Oₓ + SiO₂) und DE-A 24 40 329 (Mo₁₂BiCo_{4,5}Ni_{2,5}Cr₃P_{0,5}K_{0,1}Oₓ).

Die Stöchiometrie der Aktivmasse einer Vielzahl der für die Oxidehydrierung geeigneten Multimetalloxidkatalysatoren lässt sich unter die allgemeine Formel (I)

Mo₁₂BiₐFe_{b}Co_{c}Ni_{d}CrₑX¹_{f}K_{g}Oₓ (I),

in der die Variablen nachfolgende Bedeutung aufweisen:

| | |
|---|---|
| X¹ = | W, Sn, Mn, La, Ce, Ge, Ti, Zr, Hf, Nb, P, Si, Sb, Al, Cd und/oder Mg; |
| a = | 0,5 bis 5, vorzugsweise 0,5 bis 2; |
| b = | 0 bis 5, vorzugsweise 2 bis 4; |
| c = | 0 bis 10, vorzugsweise 3 bis 10; |
| d = | 0 bis 10; |
| e = | 0 bis 10, vorzugsweise 0,1 bis 4; |
| f = | 0 bis 5, vorzugsweise 0,1 bis 2; |
| g = | 0 bis 2, vorzugsweise 0,01 bis 1; und |
| x = | eine Zahl, die durch die Wertigkeit und Häufigkeit der von Sauerstoff verschiedenen Elemente in (I) bestimmt wird; |

subsumieren.

Bevorzugt setzt man beim erfindungsgemäßen Verfahren für die Oxidehydrierung ein Mo-Bi-Fe-O-haltiges Multimetalloxidsystem ein, wobei ein Mo-Bi-Fe-Cr-O- oder Mo-Bi-Fe-Zr-O-haltiges Multimetalloxidsystem besonders bevorzugt ist. Bevorzugte Systeme sind beispielsweise beschrieben in US 4,547,615 (Mo₁₂BiFe_{0,1}Ni₈ZrCr₃K_{0,2}Oₓ und Mo₁₂BiFe_{0,1}Ni₈AlCr₃K_{0,2}Oₓ), US 4,424,141 (Mo₁₂BiFe₃CO_{4,5}Ni_{2,5}P_{0,5}K_{0,1}Oₓ + SiO₂), DE-A 25 30 959 (Mo₁₂BiFe₃Co_{4,5}Ni_{2,5}Cr_{0,5}K_{0,1}Oₓ, Mo₁₃,₇₅BiFe₃Co_{4,5}Ni_{2,5}Ge_{0,5}K_{0,8}Oₓ, Mo₁₂BiFe₃Co_{4,5}Ni_{2,5}Mn_{0,5}K_{0,1}Ox und Mo₁₂BiFe₃Co_{4,5}Ni_{2,5}La_{0,5}K_{0,1}Oₓ), US 3,911,039 (Mo₁₂BiFe₃Co_{4,5}Ni_{2,5}Sn_{0,5}K_{0,1}Oₓ), DE-A 25 30 959 und DE-A 24 47 825 (Mo₁₂BiFe₃Co_{4,5}Ni_{2,5}W_{0,5}K_{0,1}Oₓ). Herstellung und Charakterisierung der genannten Katalysatoren sind ausführlich in den zitierten Schriften beschrieben.

Der Katalysator zur Oxidehydrierung wird im Allgemeinen als Formkörper mit einer mittleren Größe von über 2 mm eingesetzt. Aufgrund des zu beachtenden Druckverlustes während der Ausübung des Verfahrens sind kleinere Formkörper in der Regel ungeeignet. Als geeignete Formkörper seien beispielsweise genannt Tabletten, Zylinder, Hohlzylinder, Ringe, Kugeln, Stränge, Wagenräder oder Extrudate. Besondere Formen, wie beispielsweise "Trilobes" und "Tristars" (siehe EP-A-0 593 646) oder Formkörper mit mindestens einer Einkerbung an der Außenseite (siehe US 5,168,090) sind ebenfalls möglich.

Im Allgemeinen kann der verwendete Katalysator als so genannter Vollkatalysator eingesetzt werden. In diesem Fall besteht der gesamte Katalysatorformkörper aus der Aktivmasse, inklusive eventueller Hilfsmittel, wie etwa Graphit oder Porenbildner, sowie weiterer Komponenten. Insbesondere hat es sich als günstig erwiesen, den für die Oxidehydrierung der n-Butene zu Butadien bevorzugt eingesetzten Mo-Bi-Fe-O-haltigen Katalysator als Vollkatalysator einzusetzen. Des Weiteren ist es möglich, die Aktivmassen der Katalysatoren auf einen Träger, beispielsweise einen anorganischen, oxidischen Formkörper, aufzubringen. Derartige Katalysatoren werden in der Regel als Schalenkatalysatoren bezeichnet.

Die Oxidehydrierung wird im Allgemeinen bei einer Temperatur von 220 bis 490 °C, bevorzugt von 250 bis 450 °C und besonders bevorzugt von 300 bis 350 °C durchgeführt. Man wählt einen Reaktoreingangsdruck, der ausreicht, die in der Anlage und der nachfolgenden Aufarbeitung vorhandenen Strömungswiderstände zu überwinden. Dieser Reaktoreingangsdruck liegt in der Regel bei 0,005 bis 1 MPa Überdruck, bevorzugt 0,01 bis 0,5 MPa Überdruck. Naturgemäß fällt der im Eingangsbereich des Reaktors angewendete Gasdruck weitgehend über die gesamte Schüttung aus Katalysator ab.

Durch die Kopplung der nicht-oxidativen katalytischen, bevorzugt autothermen Dehydrierung mit der oxidativen Dehydrierung der gebildeten n-Butene wird eine sehr viel höhere Ausbeute an Butadien, bezogen auf eingesetztes n-Butan, erhalten. Ferner kann die nicht-oxidative Dehydrierung schonender betrieben werden. Vergleichbare Butadien-Ausbeuten wären mit einer ausschließlich nicht-oxidativen Dehydrierung nur zum Preis deutlich niedrigerer Selektivitäten zu erreichen. Mit einer ausschließlich oxidativen Dehydrierung würden nur geringe n-Butan-Umsätze erzielt werden.

Der die oxidative Dehydrierung verlassende Produktgasstrom c enthält neben Butadien und nicht umgesetztem n-Butan noch 2-Buten und Wasserdampf. Als Nebenbestandteile enthält er im Allgemeinen Kohlenmonoxid, Kohlendioxid, Sauerstoff, Stickstoff, Methan, Ethan, Ethen, Propan und Propen, gegebenenfalls Wasserstoff sowie sauerstoffhaltige Kohlenwasserstoffe, so genannte Oxygenate. Im Allgemeinen enthält er nur noch geringe Anteile an 1-Buten.

Im Allgemeinen weist der die oxidative Dehydrierung verlassende Produktgasstrom c 1 bis 40 Vol.-% Butadien, 20 bis 80 Vol.-% n-Butan, 0,5 bis 40 Vol.-% 2-Buten, 0 bis 5 Vol.% 1-Buten, 0 bis 70 Vol.-% Wasserdampf, 0 bis 10 Vol.% leichtsiedende Kohlenwasserstoffe (Methan, Ethan, Ethen, Propan und Propen), 0,1 bis 40 Vol.-% Wasserstoff, 0 bis 70 Vol.-% Stickstoff, 0 bis 10 Vol.% Kohlenstoffoxide und 0 bis 10 Vol.% Oxygenate auf. Oxygenate können beispielsweise Furan, Essigsäure, Maleinsäureanhydrid, Ameisensäure und Butyraldehyd sein.

In einem Verfahrensteil D werden die von den C₄-Kohlenwasserstoffen (n-Butan, isoButan, 1-Buten, cis-/trans-2-Buten, iso-Buten, Butadien) verschiedenen leicht siedenden Nebenbestandteile zumindest teilweise, vorzugsweise aber im wesentlichen vollständig aus dem Produktgasstrom der n-Butan-Dehydrierung abgetrennt, wobei ein C₄-Produktgasstrom d erhalten wird.

In einer Ausführungsform des erfindungsgemäßen Verfahrens wird im Verfahrensteil D zunächst Wasser aus dem Produktgasstrom c abgetrennt. Die Abtrennung von Wasser kann beispielsweise durch Auskondensieren durch Abkühlen und/oder Verdichten des Produktgasstroms c erfolgen und kann in einer oder mehreren Abkühlungs- und/oder Verdichtungsstufen durchgeführt werden.

Die Abtrennung der leicht siedenden Nebenbestandteile aus dem Produktgasstrom kann durch übliche Trennverfahren wie Destillation, Rektifikation, Membranverfahren, Absorption oder Adsorption erfolgen.

Zur Abtrennung des im Produktgasstrom c enthaltenen Wasserstoffs kann das Produktgasgemisch, gegebenenfalls nach erfolgter Kühlung, beispielsweise in einem indirekten Wärmetauscher, über eine in der Regel als Rohr ausgebildete Membran geleitet werden, die lediglich für molekularen Wasserstoff durchlässig ist. Der so abgetrennte molekulare Wasserstoff kann bei Bedarf zumindest teilweise in der Dehydrierung eingesetzt oder aber einer sonstigen Verwertung zugeführt werden, beispielsweise zur Erzeugung elektrischer Energie in Brennstoffzellen eingesetzt werden.

Das in dem Produktgasstrom c enthaltene Kohlendioxid kann durch CO₂-Gaswäsche abgetrennt werden. Der Kohlendioxid-Gaswäsche kann eine gesonderte Verbrennungsstufe vorgeschaltet werden, in der Kohlenmonoxid selektiv zu Kohlendioxid oxidiert wird.

In einer bevorzugten Ausführungsform des erfindungsgemäßen Verfahrens werden die nicht kondensierbaren oder leicht siedenden Gasbestandteile wie Wasserstoff, Sauerstoff, Kohlenstoffoxide, die leicht siedenden Kohlenwasserstoffe (Methan, Ethan, Ethen, Propan, Propen) und gegebenenfalls Stickstoff in einem Absorptions-/Desorptions-Cyclus mittels eines hoch siedenden Absorptionsmittels abgetrennt, wobei ein C₄-Produktgasstrom c erhalten wird, der im Wesentlichen aus den C₄-Kohlenwasserstoffen besteht. Im Allgemeinen besteht der C₄-Produktgasstrom c zu mindestens 80 Vol.-%, bevorzugt zu mindestens 90 Vol.-%, besonders bevorzugt zu mindestens 95 Vol.% aus den C₄-Kohlenwasserstoffen. Im Wesentlichen besteht der Strom d aus n-Butan, 2-Buten und Butadien.

Dazu wird in einer Absorptionsstufe der Produktgasstrom c nach vorheriger Wasserabtrennung mit einem inerten Absorptionsmittel in Kontakt gebracht und werden die C₄-Kohlenwasserstoffe in dem inerten Absorptionsmittel absorbiert, wobei mit C₄-Kohlenwasserstoffen beladenes Absorptionsmittel und ein die übrigen Gasbestandteile enthaltendes Abgas erhalten werden. In einer Desorptionsstufe werden die C₄-Kohlenwasserstoffe aus dem Absorptionsmittel wieder freigesetzt.

In der Absorptionsstufe eingesetzte inerte Absorptionsmittel sind im Allgemeinen hochsiedende unpolare Lösungsmittel, in denen das abzutrennende C₄-KohlenwasserstoffGemisch eine deutlich höhere Löslichkeit als die übrigen abzutrennenden Gasbestandteile aufweist. Die Absorption kann durch einfaches Durchleiten des Produktgasstroms c durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Geeignete Absorptionskolonnen sind z.B. Bodenkolonnen mit Glocken-, Zentrifugal- und/oder Siebböden, Kolonnen mit strukturierten Packungen, z.B. Blechpackungen mit einer spezifischen Oberfläche von 100 bis 1000 m²/m³ wie Mellapak^{®} 250 Y, und Füllkörperkolonnen. Es kommen aber auch Riesel- und Sprühtürme, Graphitblockabsorber, Oberflächenabsorber wie Dickschicht- und Dünnschichtabsorber sowie Rotationskolonnen, Tellerwäscher, Kreuzschleierwäscher und Rotationswäscher in Betracht.

Geeignete Absorptionsmittel sind vergleichsweise unpolare organische Lösungsmittel, beispielsweise aliphatische C₈- bis C₁₈-Alkene, oder aromatische Kohlenwasserstoffe wie die Mittelölfraktionen aus der Paraffindestillation, oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁-C₈-Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl^{®}. Häufig enthält dieses Lösungsmittelgemisch Dimethylphthalat in einer Menge von 0,1 bis 25 Gew.%. Geeignete Absorptionsmittel sind ferner Octane, Nonane, Decane, Undecane, Dodecane, Tridecane, Tetradecane, Pentadecane, Hexadecane, Heptadecane und Octadecane oder aus Raffinerieströmen gewonnene Fraktionen, die als Hauptkomponenten die genannten linearen Alkane enthalten.

Zur Desorption der C₄-Kohlenwasserstoffe wird das beladene Absorptionsmittel erhitzt und/oder auf einen niedrigeren Druck entspannt. Alternativ dazu kann die Desorption auch durch Strippung oder in einer Kombination von Entspannung, Erhitzen und Strippung in einem oder mehreren Verfahrensschritten erfolgen. Das in der Desorptionsstufe regenerierte Absorptionsmittel wird in die Absorptionsstufe zurückgeführt.

In einer Verfahrensvariante wird der Desorptionsschritt durch Entspannung und/oder Erhitzen des beladenen Desorptionsmittels durchgeführt.

Die Abtrennung D ist im Allgemeinen nicht ganz vollständig, so dass in dem C₄-Produktgasstrom - je nach Art der Abtrennung - noch geringe Mengen oder auch nur Spuren der weiteren Gasbestandteile, insbesondere der leicht siedenden Kohlenwasserstoffe, vorliegen können.

In einem Verfahrensteil E wird der C₄-Produktgasstrom d in eine Destillationszone eingespeist und in einen Wertproduktstrom e aus einem Butadien/Butan-Azeotrop und einen Strom e2, der im Wesentlichen aus n-Butan und 2-Buten besteht, aufgetrennt.

Die Destillationszone besteht im Allgemeinen aus einer Destillationskolonne mit im Allgemeinen 30 bis 80, bevorzugt 40 bis 75 theoretischen Trennstufen. Geeignet sind beispielsweise Glockenbodenkolonnen, Füllkörperkolonnen, Packungskolonnen oder Trennwandkolonnen. Das Rücklaufverhältnis beträgt im Allgemeinen 10 bis 50. Die Destillation wird im Allgemeinen bei einem Druck von 5 bis 20 x 10⁵ Padurchgeführt.

Im oberen Teil der Kolonne, vorzugsweise am Kolonnenkopf, wird ein Butadien/n-Butan-Gemisch e abgezogen. Das Butadien/n-Butan-Gemisch kann die Zusammensetzung des Azeotrops aufweisen oder einen geringeren Butadiengehalt aufweisen, im Allgemeinen enthält das Butadien/n-Butan-Gemisch mindestens 60 Vol.-% Butadien.

Der so generierte Wertproduktstrom e, der 1,3-Butadien und n-Butan enthält, kann in einer Hydrocyanierung von 1,3-Butadien eingesetzt werden.

Dabei ist es nicht zwingend erforderlich, dass das 1,3-Butadien stabilisiert wird.

Als Hydrocyanierungskatalysator wird vorzugsweise ein homogener Nickel(0)-Katalysator verwendet, der mit Phosphorliganden stabilisiert ist.

Die phosphorhaltigen Liganden der Nickel(0)-Komplexe und die freien phosphorhaltigen Ligandensind vorzugsweise ausgewählt aus mono- oder bidentaten Phosphinen, Phosphiten, Phosphiniten und Phosphoniten.

Diese phosphorhaltigen Liganden weisen vorzugsweise die Formel I auf:

P (X¹R¹) (X²R²) (X³R³) (I)

Unter Verbindung I wird im Sinne der vorliegenden Erfindung eine einzelne Verbindung oder ein Gemisch verschiedener Verbindungen der vorgenannten Formel verstanden.

Erfindungsgemäß sind X¹, X², X³ unabhängig voneinander Sauerstoff oder Einzeibindung. Falls alle der Gruppen X¹, X² und X³ für Einzelbindungen stehen, so stellt Verbindung I ein Phosphin der Formel P(R¹R²R³) mit den für R¹, R² und R³ in dieser Beschreibung genannten Bedeutungen dar.

Falls zwei der Gruppen X¹, X² und X³ für Einzelbindungen stehen und eine für Sauerstoff, so stellt Verbindung I ein Phosphinit der Formel P(OR¹)(R²)(R³) oder

Falls zwei der Gruppen X¹, X² und X³ für Einzelbindungen stehen und eine für Sauerstoff, so stellt Verbindung I ein Phosphinit der Formel P(OR¹)(R²)(R³) oder P(R¹)(OR²)(R³) oder P(R¹)(R²)(OR³) mit den für R¹, R² und R³ weiter unten genannten Bedeutungen dar.

Falls eine der Gruppen X¹, X² und X³ für eine Einzelbindung steht und zwei für Sauerstoff, so stellt Verbindung I ein Phosphonit der Formel P(OR¹)(OR²)(R³) oder P(R¹)(OR²)(OR³) oder P(OR¹)(R²)(OR³) mit den für R¹, R² und R³ in dieser Beschreibung genannten Bedeutungen dar.

In einer bevorzugten Ausführungsform sollten alle der Gruppen X¹, X² und X³ für Sauerstoff stehen, so dass Verbindung I vorteilhaft ein Phosphit der Formel P(OR¹)(OR²)(OR³) mit den für R¹, R² und R³ weiter unten genannten Bedeutungen darstellt.

Erfindungsgemäß stehen R¹, R², R³ unabhängig voneinander für gleiche oder unterschiedliche organische Reste. Als R¹, R² und R³ kommen unabhängig voneinander Alkylreste, vorzugsweise mit 1 bis 10 Kohlenstoffatomen, wie Methyl, Ethyl, n-Propyl, i-Propyl, n-Butyl, i-Butyl, s-Butyl, t-Butyl, Aryl-Gruppen, wie Phenyl, o-Tolyl, m-Tolyl, p-Tolyl, 1-Naphthyl, 2-Naphthyl, oder Hydrocarbyl, vorzugsweise mit 1 bis 20 Kohlenstoffatomen, wie 1,1'-Biphenol, 1,1'-Binaphthol in Betracht. Die Gruppen R¹, R² und R³ können miteinander direkt, also nicht allein über das zentrale Phosphor-Atom, verbunden sein. Vorzugsweise sind die Gruppen R¹, R² und R³ nicht miteinander direkt verbunden.

In einer bevorzugten Ausführungsform kommen als Gruppen R¹, R² und R³ Reste ausgewählt aus der Gruppe bestehend aus Phenyl, o-Tolyl, m-Tolyl und p-Tolyl in Betracht. In einer besonders bevorzugten Ausführungsform sollten dabei maximal zwei der Gruppen R¹, R² und R³ Phenyl-Gruppen sein.

In einer anderen bevorzugten Ausführungsform sollten dabei maximal zwei der Gruppen R¹, R² und R³ o-Tolyl-Gruppen sein.

Als besonders bevorzugte Verbindungen I können solche der Formel I a

(o-Tolyl-O-)_{w} (m-Tolyl-O-)ₓ (p-Tolyl-O-)_{y} (Phenyl-O-)_{z} P (I a)

eingesetzt werden, wobei w, x, y und z eine natürliche Zahl bedeuten, und folgende Bedingungen gelten: w + x + y + z = 3 und w, z ≤ 2.

Solche Verbindungen I a sind z.B. (p-Tolyl-O-)(Phenyl-O-)₂P, (m-Tolyl-O-)(Phenyl-O-)₂P, (o-Tolyl-O-) (Phenyl-O-)₂P, (p-Tolyl-O-)₂(Phenyl-O-)P, (m-Tolyl-O-)₂(Phenyl-O-)P, (o-Tolyl-O-)₂(Phenyl-O-)P, (m-Tolyl-O-)(p-Tolyl-O)(Phenyl-O-)P, (o-Tolyl-O-)(p-Tolyl-O-)(Phenyl-O-)P, (o-Tolyl-O-)(m-Tolyl-O-)(Phenyl-O-)P, (p-Tolyt-O-)₃P, (m-Tolyl-O-)(p-Tolyl-O-)₂P, (o-Tolyl-O-)(p-Tolyl-O-)₂P, (m-Tolyl-O-)₂(p-Toluyl-O-)P, (o-Tolyl-O-)₂(p-Tolyl-O-)P, (o-Tolyl-O-)(m-Tolyl-O-)(p-Tolyl-O)P, (m-Tolyl-O-)₃P, (o-Tolyl-O-)(m-Tolyl-O-)₂P (o-Tolyl-O-)₂(m-Tolyl-O-)P, oder Gemische solcher Verbindungen.

Gemische enthaltend (m-Tolyl-O-)₃P, (m -Tolyl-O-)₂(p-Tolyl-O-)P, (m-Tolyl-O-)(p-Tolyl-O-)₂P und (p-Tolyl-O-)₃P kann man beispielsweise durch Umsetzung eines Gemisches enthaltend m-Kresol und p-Kresol, insbesondere im Molverhältnis 2: 1, wie es bei der destillativen Aufarbeitung von Erdöl anfällt, mit einem Phosphortrihalogenid, wie Phosphortrichlorid, erhalten.

In einer anderen, ebenfalls bevorzugten Ausführungsform kommen als phosphorhaltige Liganden die in der DE-A 199 53 058 näher beschriebenen Phosphite der Formel I b in Betracht:

P (O-R¹)ₓ (O-R²)_{y} (O-R³)_{z}, (O-R⁴)ₚ (I b)

mit
- R¹:: aromatischer Rest mit einem C₁-C₁₈-Alkylsubstituenten in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromatischen System verbindet, anellierten aromatischen System,
- R²: aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoffatom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der aromatische Rest in o-Stellung zu dem Sauerstoffatom, das das Phosphoratom mit dem aromatischen System verbindet, ein Wasserstoffatom trägt,
- R³:: aromatischer Rest mit einem C₁-C₁₈-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aromatische Rest in o-Stellung zu dem Sauerstoffatom, das das Phosphoratom mit dem aromatischen System verbindet, ein Wasserstoffatom trägt,
- R⁴:: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt,
- x :: 1 oder 2,
- y, z, p:: unabhängig voneinander 0, 1 oder 2 mit der Maßgabe, dass x+y+z+p = 3.

Bevorzugte Phosphite der Formel I b sind der DE-A 199 53 058 zu entnehmen. Als Rest R¹ kommen vorteilhaft o-Tolyl-, o-Ethyl-phenyl-, o-n-Propyl-phenyl-, o-Isopropyl-phenyl-, o-n-Butyl-phenyl-, o-sek-Butyl-phenyl-, o-tert-Butyl-phenyl-, (o-Phenyl)-Phenyl- oder 1-Naphthyl- Gruppen in Betracht.

Als Rest R² sind m-Tolyl-, m-Ethyl-phenyl-, m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen bevorzugt.

Als Rest R³ kommen vorteilhaft p-Tolyl-, p-Ethyl-phenyl-, p-n-Propyl-phenyl-, p-Isopropyl-phenyl-, p-n-Butyl-phenyl-, p-sek-Butyl-phenyl-, p-tert-Butyl-phenyl- oder (p-Phenyl)-Phenyl-Gruppen in Betracht.

Rest R⁴ ist bevorzugt Phenyl. Vorzugsweise ist p gleich null. Für die Indizes x, y, z und p in Verbindung I b ergeben sich folgende Möglichkeiten:

| x | y | z | p |
|---|---|---|---|
| 1 | 0 | 0 | 2 |
| 1 | 0 | 1 | 1 |
| 1 | 1 | 0 | 1 |
| 2 | 0 | 0 | 1 |
| 1 | 0 | 2 | 0 |
| 1 | 1 | 1 | 0 |
| 1 | 2 | 0 | 0 |
| 2 | 0 | 1 | 0 |
| 2 | 1 | 0 | 0 |

Bevorzugte Phosphite der Formel I b sind solche, in denen p gleich null ist sowie R¹, R² und R³ unabhängig voneinander ausgewählt sind aus o-Isopropyl-phenyl, m-Tolyl und p-Tolyl, und R⁴ Phenyl ist.

Besonders bevorzugte Phosphite der Formel I b sind solche, in denen R¹ der o-Isopropyl-phenyl-Rest, R² der m-Tolylrest und R³ der p-Tolylrest ist mit den in der vorstehenden Tabelle genannten Indizes; außerdem solche, in denen R¹ der o-Tolylrest, R² der m-Tolylrest und R³ der p-Tolylrest ist mit den in der Tabelle genannten Indizes; weiterhin solche, in denen R¹ der 1-Naphthylrest, R² der m-Tolylrest und R³ der p-Tolylrest ist mit den in der Tabelle genannten Indizes; außerdem solche, in denen R¹ der o-Tolylrest, R² der 2-Naphthylrest und R³ der p-Tolylrest ist mit den in der Tabelle genannten Indizes; und schließlich solche, in denen R¹ der o-Isopropyl-phenyl-Rest, R² der 2-Naphthylrest und R³ der p-Tolylrest ist mit den in der Tabelle genannten Indizes; sowie Gemische dieser Phosphite.

Phosphite der Formel I b können erhalten werden, indem man
a) ein Phosphortrihalogenid mit einem Alkohol ausgewählt aus der Gruppe bestehend aus R¹OH, R²OH, R³OH und R⁴OH oder deren Gemische umsetzt unter Erhalt eines Dihalogenophosphorigsäuremonoesters,
b) den genannten Dihalogenophosphorigsäuremonoester mit einem Alkohol ausgewählt aus der Gruppe bestehend aus R¹ OH, R² OH, R³ OH und R⁴OH oder deren Gemische umsetzt unter Erhalt eines Monohalogenophosphorigsäurediesters und
c) den genannten Monohalogenophosphorigsäurediester mit einem Alkohol ausgewählt aus der Gruppe bestehend aus R¹ OH, R²OH, R³ OH und R⁴OH oder deren Gemische umsetzt unter Erhalt eines Phosphits der Formel I b.

Die Umsetzung kann in drei getrennten Schritten durchgeführt werden. Ebenso können zwei der drei Schritte kombiniert werden, also a) mit b) oder b) mit c). Alternativ können alle der Schritte a), b) und c) miteinander kombiniert werden.

Dabei kann man geeignete Parameter und Mengen der Alkohole ausgewählt aus der Gruppe bestehend aus R¹OH, R²OH, R³OH und R⁴OH oder deren Gemische durch einige einfache Vorversuche leicht ermitteln.

Als Phosphortrihalogenid kommen grundsätzlich alle Phosphortrihalogenide, vorzugsweise solche, in denen als Halogenid Cl, Br, I, insbesondere Cl, eingesetzt wird; sowie deren Gemische in Betracht. Es können auch Gemische verschiedener gleich oder unterschiedlich halogensubstituierter Phosphine als Phosphortrihalogenid eingesetzt werden. Besonders bevorzugt ist PCl₃. Weitere Einzelheiten zu den Reaktionsbedingungen bei der Herstellung der Phosphite I b und zur Aufarbeitung sind der DE-A 199 53 058 zu entnehmen.

Die Phosphite I b können auch in Form eines Gemisches verschiedener Phosphite I b als Ligand verwendet werden. Ein solches Gemisch kann beispielsweise bei der Herstellung der Phosphite I b anfallen.

Es ist allerdings bevorzugt, dass der phosphorhaltige Ligand mehrzähnig, insbesondere zweizähnig ist. Daher weist der verwendete Ligand vorzugsweise die Formel II 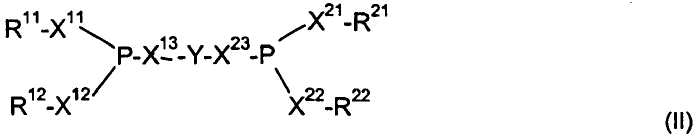 auf, worin bedeuten
- X¹¹, X¹², X¹³, X²¹, X²², X²³: unabhängig voneinander Sauerstoff oder Einzelbindung
- R¹¹, R¹²: unabhängig voneinander gleiche oder unterschiedliche, einzelne oder verbrückte organische Reste
- R²¹ , R²²: unabhängig voneinander gleiche oder unterschiedliche, einzelne oder verbrückte organische Reste,
- Y: Brückengruppe

Unter Verbindung II wird im Sinne der vorliegenden Erfindung eine einzelne Verbindung oder ein Gemisch verschiedener Verbindungen der vorgenannten Formel verstanden.

In einer bevorzugten Ausführungsform können X¹¹, X¹², X¹³, X²¹, X²², X²³ Sauerstoff darstellen. In einem solchen Fall ist die Brückengruppe Y mit Phosphit-Gruppen verknüpft.

In einer anderen bevorzugten Ausführungsform können X¹¹ und X¹² Sauerstoff und X¹³ eine Einzelbindung oder X¹¹ und X¹³ Sauerstoff und X¹² eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²¹ und X²² Sauerstoff und X²³ eine Einzelbindung oder X²¹ und X²³ Sauerstoff und X²² eine Einzelbindung oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphonits, Phosphinits oder Phosphins, vorzugsweise eines Phosphonits, sein kann.

In einer anderen bevorzugten Ausführungsform können X¹³ Sauerstoff und X¹¹ und X¹² eine Einzeibindung oder X¹¹ Sauerstoff und X¹² und X¹³ eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphinits oder Phosphins, vorzugsweise eines Phosphinits, sein kann.

In einer anderen bevorzugten Ausführungsform können X¹¹, X¹² und X¹³ eine Einzelbindung darstellen, so dass das mit X¹¹, X¹² und X¹³ umgebene Phosphoratom Zentralatom eines Phosphins ist. In einem solchen Fall können X²¹, X²² und X²³ Sauerstoff oder X²¹, X²² und X²³ eine Einzelbindung darstellen, so dass das mit X²¹, X²² und X²³ umgebene Phosphoratom Zentralatom eines Phosphits oder Phosphins, vorzugsweise eines Phosphins, sein kann.

Als Brückengruppe Y kommen vorzugsweise substituierte, beispielsweise mit C₁-C₄-Alkyl, Halogen, wie Fluor, Chlor, Brom, halogeniertem Alkyl, wie Trifluormethyl, Aryl, wie Phenyl, oder unsubstituerte Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste R¹¹ und R¹² können unabhängig voneinander gleiche oder unterschiedliche organische Reste darstellen. Vorteilhaft kommen als Reste R¹¹ und R¹² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R²¹ und R²² können unabhängig voneinander gleiche oder unterscheidliche organische Reste darstellen. Vorteilhaft kommen als Reste R²¹ und R²² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R¹¹ und R¹² können einzeln oder verbrückt sein. Auch die Reste R²¹ und R²² können einzeln oder verbrückt sein. Die Reste R¹¹, R¹², R²¹ und R²² können alle einzeln, zwei verbrückt und zwei einzeln oder alle vier verbrückt sein in der beschriebenen Art.

In einer besonders bevorzugten Ausführungsform kommen die in US 5,723,641 genannten Verbindungen der Formel I, II, III, IV und V in Betracht. In einer besonders bevorzugten Ausführungsform kommen die in US 5,512,696 genannten Verbindungen der Formel I, II, III IV, V, VI und VII, insbesondere die dort in den Beispielen 1 bis 31 eingesetzten Verbindungen, in Betracht. In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzten Verbindungen, in Betracht.

In einer besonders bevorzugten Ausführungsform kommen die in US 5,512,695 genannten Verbindungen der Formel I, II, III, IV, V und VI, insbesondere die dort in den Beispielen 1 bis 6 eingesetzten Verbindungen, in Betracht. In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzten Verbindungen, in Betracht.

In einer besonders bevorzugten Ausführungsform kommen die in US 6,127,567 genannten Verbindungen und dort in den Beispielen 1 bis 29 eingesetzten Verbindungen in Betracht. In einer besonders bevorzugten Ausführungsform kommen die in US 6,020,516 genannten Verbindungen der Formel I, II, III, IV, V, VI, VII, VIII, IX und X, insbesondere die dort in den Beispielen 1 bis 33 eingesetzten Verbindungen, in Betracht. In einer besonders bevorzugten Ausführungsform kommen die in US 5,959,135 genannten Verbindungen und dort in den Beispielen 1 bis 13 eingesetzten Verbindungen in Betracht.

In einer besonders bevorzugten Ausführungsform kommen die in US 5,847,191 genannten Verbindungen der Formel I, II und III in Betracht. In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 13, 14, 15, 16, 17, 18, 19, 20 und 21 dargestellten Verbindungen, in Betracht. In einer besonders bevorzugten Ausführungsform kommen die in WO 01/14392 genannten Verbindungen, vorzugsweise die dort in Formel V, VI, VII, VIII, IX, X, XI, XII, XIII, XIV, XV, XVI, XVII, XXI, XXII, XXIII dargestellten Verbindungen, in Betracht.

In einer besonders bevorzugten Ausführungsform kommen die in WO 98/27054 genannten Verbindungen in Betracht. In einer besonders bevorzugten Ausführungsform kommen die in WO 99/13983 genannten Verbindungen in Betracht. In einer besonders bevorzugten Ausführungsform kommen die in WO 99/64155 genannten Verbindungen in Betracht.

In einer besonders bevorzugten Ausführungsform kommen die in der deutschen Patentanmeldung DE 100 380 37 genannten Verbindungen in Betracht. In einer besonders bevorzugten Ausführungsform kommen die in der deutschen Patentanmeldung DE 100 460 25 genannten Verbindungen in Betracht. In einer besonders bevorzugten Ausführungsform kommen die in der deutschen Patentanmeldung DE 101 502 85 genannten Verbindungen in Betracht.

In einer besonders bevorzugten Ausführungsform kommen die in der deutschen Patentanmeldung DE 101 502 86 genannten Verbindungen in Betracht. In einer besonders bevorzugten Ausführungsform kommen die in der deutschen Patentanmeldung DE 102 071 65 genannten Verbindungen in Betracht. In einer weiteren besonders bevorzugten Ausführungsform der vorliegenden Erfindung kommen die in der US 2003/0100442 A1 genannten phosphorhaltigen Chelatliganden in Betracht.

In einer weiteren besonders bevorzugten Ausführungsform der vorliegenden Erfindung kommen die in der nicht vorveröffentlichten deutschen Patentanmeldung Aktenzeichen DE 103 50 999.2 vom 30.10.2003 genannten phosphorhaltigen Chelatliganden in Betracht.

Die beschriebenen Verbindungen I, I a, I b und II sowie deren Herstellung sind an sich bekannt. Als phosphorhaltiger Ligand können auch Mischungen, enthaltend mindestens zwei der Verbindungen I, I a, I b und II, eingesetzt werden.

In einer besonders bevorzugten Ausführungsform des erfindungsgemäßen Verfahrens ist der phosphorhaltige Ligand des Nickel(0)-Komplexes und/oder der freie phosphorhaltige Ligand ausgewählt aus Tritolylphosphit, bidentaten phosphorhaltigen Chelatliganden, sowie den Phosphiten der Formel I b

P (O-R¹)ₓ (O-R²)_{y} (O-R³)_{z} (O-R⁴)ₚ (I b)

worin R¹, R² und R³ unabhängig voneinander ausgewählt sind aus o-Isopropyl-phenyl, m-Tolyl und p-Tolyl, R⁴ Phenyl ist; x gleich 1 oder 2 ist, und y, z, p unabhängig voneinander 0,.1 oder 2 sind mit der Maßgabe, dass x+y+z+p = 3 ist; und deren Mischungen.

Die Hydrocyanierung kann in jeder geeigneten, dem Fachmann bekannten Vorrichtung durchgeführt werden. Für die Reaktion kommen somit übliche Vorrichtungen in Betracht, wie sie beispielsweise in: Kirk-Othmer, Encyclopedia of Chemical Technology, 4. Ed., Vol. 20, John Wiley & Sons, New York, 1996, Seiten 1040 bis 1055 beschrieben sind, wie Rührkesselreaktoren, Schlaufenreaktoren, Gasumlaufreaktoren, Blasensäulenreaktören oder Rohrreaktoren, jeweils gegebenenfalls mit Vorrichtungen zur Abfuhr von Reaktionswärme. Die Reaktion kann in mehreren, wie zwei oder drei, Vorrichtungen durchgeführt werden.

In einer bevorzugten Ausführungsform des erfindungsgemäßen Verfahrens haben sich Reaktoren mit Rückvermischungscharakteristik oder Kaskaden von Reaktoren mit Rückvermischungscharakteristik als vorteilhaft erwiesen. Als besonders vorteilhaft haben sich Kaskaden aus Reaktoren mit Rückvermischungscharakteristik erwiesen, die in Bezug auf die Dosierung von Cyanwasserstoff in Querstromfahrweise betrieben werden.

Die Hydrocyanierung kann in Gegenwart oder in Abwesenheit von einem Lösemittel durchgeführt werden. Wenn ein Lösemittel verwendet wird, so sollte das Lösemittel bei der gegebenen Reaktionstemperatur und dem gegebenen Reaktionsdruck flüssig und inert gegenüber den ungesättigten Verbindungen und dem mindestens einen Katalysator sein. Im Allgemeinen werden als Lösemittel Kohlenwasserstoffe, beispielsweise Benzol oder Xylol, oder Nitrile, beispielsweise Acetonitril oder Benzonitril, verwendet. Vorzugsweise wird allerdings ein Ligand als Lösemittel verwendet.

Die Reaktion kann in Batchfahrweise, kontinuierlich oder im Semibatchbetrieb durchgeführt werden.

Die Hydrocyanierungsreaktion kann durchgeführt werden, indem die Vorrichtung mit allen Reaktanten bestückt wird. Bevorzugt ist allerdings, wenn die Vorrichtung mit dem Katalysator, der ungesättigten organischen Verbindung und gegebenenfalls dem Lösemittel gefüllt wird. Vorzugsweise schwebt der gasförmige Cyanwasserstoff über der Oberfläche der Reaktionsmischung oder wird durch die Reaktionsmischung geleitet. Eine weitere Verfahrensweise zum Bestücken der Vorrichtung ist das Befüllen der Vorrichtung mit dem Katalysator, Cyanwasserstoff und gegebenenfalls dem Lösemittel und das langsame Zuspeisen der ungesättigten Verbindung zu der Reaktionsmischung. Alternativ ist auch möglich, dass die Reaktanten in den Reaktor eingeführt werden und die Reaktionsmischung auf die Reaktionstemperatur gebracht wird, bei welcher der Cyanwasserstoff flüssig zu der Mischung gegeben wird. Darüber hinaus kann der Cyanwasserstoff auch vor dem Erwärmen auf Reaktionstemperatur zugegeben werden. Die Reaktion wird unter konventionellen Hydrocyanierungsbedingungen für Temperatur, Atmosphäre, Reaktionszeit, etc. durchgeführt.

Vorzugsweise wird die Hydrocyanierung kontinuierlich in einem oder mehreren gerührten Verfahrensschritten durchgeführt. Wenn eine Mehrzahl von Verfahrensschritten verwendet wird, so ist es bevorzugt, dass die Verfahrensschritte in Serie geschaltet sind. Dabei wird das Produkt von einem Verfahrensschritt direkt in den nächsten Verfahrensschritt überführt. Der Cyanwasserstoff kann direkt in den ersten Verfahrensschritt oder zwischen den einzelnen Verfahrensschritten zugeführt werden.

Wenn die Hydrocyanierung im Semibatchbetrieb durchgeführt wird, so ist es bevorzugt, dass im Reaktor die Katalysatorkomponenten und 1,3-Butadien vorgelegt werden, während Cyanwasserstoff über die Reaktionszeit hinweg in die Reaktionsmischung dosiert wird.

Die Hydrocyanierung wird vorzugsweise bei absoluten Drücken von 0,1 bis 500 MPa, besonders bevorzugt 0,5 bis 50 MPa, insbesondere 1 bis 5 MPa, durchgeführt. Die Reaktion wird vorzugsweise bei Temperaturen von 273 bis 473 K, besonders bevorzugt 313 bis 423 K, insbesondere bei 333 bis 393 K, durchgeführt. Dabei haben sich durchschnittliche mittlere Verweilzeiten der flüssigen Reaktorphase im Bereich von 0,001 bis 100 Stunden, vorzugsweise 0,05 bis 20 Stunden, besonders bevorzugt 0,1 bis 5 Stunden, jeweils pro Reaktor, als vorteilhaft erwiesen.

Die Hydrocyanierung kann in einer Ausführungsform in flüssiger Phase in Gegenwart einer Gasphase und gegebenenfalls einer festen suspendierten Phase ausgeführt werden. Dabei können die Ausgangsstoffe Cyanwasserstoff und 1,3-Butadien jeweils flüssig oder gasförmig zudosiert werden.

Die Hydrocyanierung kann in einer weiteren Ausführungsform in flüssiger Phase durchgeführt werden, wobei der Druck im Reaktor so bemessen ist, dass alle Einsatzstoffe wie 1,3-Butadien, Cyanwasserstoff und der mindestens eine Katalysator flüssig zudosiert werden und in der Reaktionsmischung in flüssiger Phase vorliegen. Dabei kann eine feste suspendierte Phase im Reaktionsgemisch vorliegen, die auch zusammen mit dem mindestens einen Katalysator zudosiert werden kann, beispielsweise bestehend aus Abbauprodukten des Katalysatorsystems, enthaltend unter anderem Nickel(II)-Verbindungen.

In dem Wertproduktstrom e, der in dem erfindungsgemäßen in einer bevorzugten oben dargestellten Ausführungsform als Eduktstrom verwendet wird, ist n-Butan enthalten. Dieses n-Butan kann nach der Hydrocyanierung von 1,3-Butadien aus dem Hydrocyanierungsaustrag abgetrennt werden und beispielsweise in die Generierung des Wertproduktstromes e zurückgeführt werden.

In der Hydrocyanierung wird gegebenenfalls nicht umgesetztes n-Butan erhalten, das in die Dehydrierung zur Gewinnung von 1,3-Butadien zurückgeführt werden.

Das erfindungsgemäße Verfahren ist mit einer Reihe von Vorteilen verbunden. So führt der Einsatz von einem Gemisch aus 1,3-Butadien und n-Butan aus der Butandehydrierung zu. einer Herabsetzung der Kosten gegenüber dem Einsatz von reinem 1,3-Butadien. Darüber hinaus kann das n-Butan, das in dem Hydrocyanierungsaustrag vorliegt, in die Dehydrierung zur Gewinnung von 1,3-Butadien zurückgeführt werden. Eine Stabilisierung des in der Mischung enthaltenen 1,3-Butadiens ist nicht notwendig. Damit wird auch der sicherheitstechnische anspruchsvolle und unumgängliche Transport des 1,3-Butadiens zu den Vorrichtungen zur Hydrocyanierung von 1,3-Butadienen durch einen unproblematischen Transport von n-Butan ersetzt.

Die vorliegende Erfindung wird anhand einiger Ausführungsbeispiele näher erläutert.

### Beispiele:

Alle Versuche werden in einer Schutzgasatmosphäre durchgeführt.

Es werden folgende Abkürzungen verwendet:
BD: 1,3-Butadien
THF: Tetrahydrofuran

### Hydrocyanierung von BD/n-Butan (90 Vol.% / 10 Vol.-%) zu 2-Methyl-3-butennitril/ 3-Pentennitril

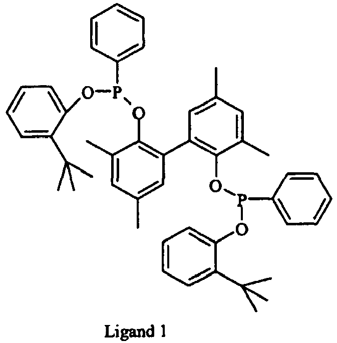

### Beispiel 1: (0,44 mmol Ni(0))

1 Äq.-Ni(COD)₂ wird mit 3 Äq. Ligand 1 in THF 20 Min. gerührt. Diese Lösung wird mit 727 Äq. BD, die als BD/n-Butan-Gemisch mit 90 Vol.% BD und 10 Vol.-% n-Butan eingesetzt werden, versetzt, bei 25 °C in einen Glausautoklaven gefüllt und auf 90 °C erwärmt. Über 60 Min. werden nun 465 Äq. Cyanwasserstoff in THF zudosiert und weitere 50 Min. bei 90 °C nachgerührt. Man bestimmt nach 110 Min das Verhältnis 2-Methyl-3-butennitril/3-Pentennitril GC-Chromatographisch (GC-Flächenprozent). Das Verhältnis 2-Methyl-3-butennitril/3-Pentennitril beträgt 1,7/1. Der Cyanwasserstoff-Umsatz beträgt > 98 % (Titration nach Vollhard).

### Hydrocyanierung von BD/n-Butan (80 Vol.% / 20 Vol.-%) zu 2-Methyl-3-butennitril/3-Pentennitril

### Beispiel 2: (0,46 mmol Ni(0)

1 Äq.-Ni(COD)₂ wird mit 3 Äq. Ligand 1 in THF 20 Min. gerührt. Diese Lösung wird mit 727 Äq. BD, die als BD/n-Butan-Gemisch mit 80 Vol.% BD und 20 Vol.-% n-Butan eingesetzt werden, versetzt, bei 25 °C in einen Glausautoklaven gefüllt und auf 90 °C erwärmt. Über 60 Min. werden nun 465 Äq. Cyanwasserstoff in THF zudosiert und weitere 75 Min. bei 90 °C nachgerührt. Man bestimmt nach 135 Min das Verhältnis 2Methyl-3-butennitril/3-Pentennitril GC-Chromatographisch (GC-Flächenprozent). Das Verhältnis 2-Methyl-3-butennitril/3-Pentennitril beträgt 1,4/1. Der Cyanwasserstoff-Umsatz beträgt > 98 % (Titration nach Vollhard).

Die Beispiele werden unter einer Schutzgasatmosphäre, beispielsweise aus Argon, unter Feuchtigkeitsausschluss durchgeführt.

## Patentansprüche

1. Verfahren zur Hydrocyanierung von 1,3-Butadien an mindestens einem Nickel(0)-Komplex mit phosphorhaltigen Liganden als Katalysator, **dadurch gekennzeichnet, dass** das 1,3-Butadien in einer Mischung mit n-Butan von 60 bis 90 Vol.-% 1,3-Butadien und 40 bis 10 Vol.-% n-Butan eingesetzt wird und durch die folgenden Verfahrensschritte hergestellt wird:
A) Bereitstellung eines n-Butan enthaltenden Einsatzgasstroms a;
B) Einspeisung des n-Butan enthaltenden Einsatzgasstroms a in mindestens eine erste Dehydrierzone und nicht-oxidative katalytische Dehydrierung von n-Butan, wobei ein Produktgasstrom b, enthaltend n-Butan, 1-Buten, 2-Buten, 1,3-Butadien, Wasserdampf, leichtsiedende Nebenbestandteile und gegebenenfalls Wasserdampf, erhalten wird;
C) Einspeisung des Produktgasstroms b der nicht-oxidativen katalytischen Dehydrierung und eines Sauerstoff-haltigen Gases in mindestens eine zweite Dehydrierzone und oxidative Dehydrierung von 1-Buten und 2-Buten, wobei ein Produktgasstrom c enthaltend n-Butan, 2-Buten, 1,3-Butadien, Wasserstoff, leichtsiedende Nebenbestandteile und Wasserdampf erhalten wird, welcher einen höheren Gehalt an 1,3-Butadien als der Produktgasstrom b aufweist;
D) Abtrennung von Wasserdampf, der leichtsiedenden Nebenbestandteile und von Wasserdampf, wobei ein C₄-Produktgasstrom d im Wesentlichen bestehend aus n-Butan, 2-Buten und 1,3-Butadien erhalten wird;
E) Einspeisung des C₄-Produktgasstroms d in eine Destillationszone und Abtrennung eines 1,3-Butadien/n-Butan-Gemisches als Wertproduktstrom e.

2. Verfahren nach Anspruch 1, **dadurch gekennzeichnet, dass** die nicht-oxidative katalytische Dehydrierung von n-Butan autotherm durchgeführt wird.

3. Verfahren nach Anspruch 1 oder 2, **dadurch gekennzeichnet, dass** der n-Butan enthaltende Einsatzgasstrom aus liquefid petroleum gas (LPG) gewonnen wird.

4. Verfahren nach einem der Ansprüche 1 bis 3, **dadurch gekennzeichnet, dass** der Nickel(0)-Katalysator phosphorhaltige Liganden umfasst, die ausgewählt sind aus der Gruppe, bestehend aus mono- oder bidentaten Phosphinen, Phosphiten, Phosphiniten, Phosphoniten und Phosphinitphosphoniten.

## Claims

1. A process for hydrocyanating 1,3-butadiene over at least one nickel(0) complex having phosphorus ligands as a catalyst, which comprises using the 1,3-butadiene in a mixture with n-butane of from 60 to 90% by volume of 1,3-butadiene and from 40 to 10% by volume of n-butane and preparing it by the following process steps:
A) providing a feed gas stream a comprising n-butane;
B) feeding the feed gas stream a comprising n-butane into at least one first dehydrogenation zone and nonoxidatively catalytically dehydrogenating n-butane to obtain a product gas stream b comprising n-butane, 1-butene, 2butene, 1,3-butadiene, steam, low-boiling secondary constituents and in some cases steam;
C) feeding the product gas stream b of the nonoxidative catalytic dehydrogenation and an oxygenous gas into at least one second dehydrogenation zone and oxidatively dehydrogenating 1-butene and 2-butene to obtain a product gas stream c comprising n-butane, 2-butene, 1,3-butadiene, hydrogen, low-boiling secondary constituents and steam, said product gas stream c having a higher content of 1,3-butadiene than the product gas stream b;
D) removing steam, low-boiling secondary constituents and steam to obtain a C₄ product gas stream d substantially consisting of n-butane, 2-butene and 1,3-butadiene;
E) feeding the C₄ product gas stream d into a distillation zone and removing a 1,3-butadiene/n-butane mixture as a product of value stream e.

2. The process according to claim 1, wherein the nonoxidative catalytic dehydrogenation of n-butane is carried out autothermally.

3. The process according to claim 1 or 2, wherein the feed gas stream comprising n-butane is obtained from liquefied petroleum gas (LPG).

4. The process according to any of claims 1 to 3, wherein the nickel(0) catalyst comprises phosphorus ligands which are selected from the group consisting of mono- or bidentate phosphines, phosphites, phosphinites, phosphonites and phosphinite phosphonites.

## Revendications

1. Procédé d'hydrocyanation de 1,3-butadiène sur au moins un complexe de nickel (0) comprenant des ligands contenant du phosphore en tant que catalyseur, **caractérisé en ce que** le 1,3-butadiène est utilisé dans un mélange avec du n-butane de 60 à 90 % en volume de 1,3-butadiène et 40 à 10 % en volume de n-butane, et est fabriqué par les étapes de procédé suivantes :
A) la préparation d'un courant gazeux d'entrée a contenant du n-butane ;
B) l'introduction du courant gazeux d'entrée a contenant du n-butane dans au moins une première zone de déshydrogénation et la déshydrogénation catalytique non oxydative du n-butane, un courant gazeux de produits b contenant du n-butane, du 1-butène, du 2-butène, du 1,3-butadiène, de la vapeur d'eau, des constituants secondaires de point d'ébullition faible et éventuellement de la vapeur d'eau étant obtenu ;
C) l'introduction du courant gazeux de produits b de la déshydrogénation catalytique non oxydative et d'un gaz contenant de l'oxygène dans au moins une seconde zone de déshydrogénation et la déshydrogénation oxydative du 1-butène et du 2-butène, un courant gazeux de produits c contenant du n-butane, du 2-butène, du 1,3-butadiène, de l'hydrogène, des constituants secondaires de point d'ébullition faible et de la vapeur d'eau étant obtenu, qui présente une teneur en 1,3-butadiène plus élevée que le courant gazeux de produits b ;
D) la séparation de la vapeur d'eau, des constituants secondaires de point d'ébullition faible et de la vapeur d'eau, un courant gazeux de produits en C₄ d essentiellement constitué de n-butane, de 2-butène et de 1,3-butadiène étant obtenu ;
E) l'introduction du courant gazeux de produits en C₄ d dans une zone de distillation et la séparation d'un mélange 1,3-butadiène/n-butane en tant que courant de produits de valeur e.

2. Procédé selon la revendication 1, **caractérisé en ce que** la déshydrogénation catalytique non oxydative du n-butane est réalisée de manière autotherme.

3. Procédé selon la revendication 1 ou 2, **caractérisé en ce que** le courant gazeux d'entrée contenant du n-butane est obtenu à partir de gaz de pétrole liquéfié (GPL).

4. Procédé selon l'une quelconque des revendications 1 à 3, **caractérisé en ce que** le catalyseur de nickel (0) comprend des ligands contenant du phosphore, qui sont choisis dans le groupe constitué par les phosphines, phosphites, phosphinites, phosphonites et phosphinite-phosphonites mono- ou bidentates.
